(19) Europäisches Patentamt / European Patent Office / Office européen des brevets

(11) **EP 2 769 286 B1**

(12) **EUROPEAN PATENT SPECIFICATION**

(45) Date of publication and mention
of the grant of the patent:
**01.08.2018 Bulletin 2018/31**

(51) Int Cl.:
***G06F 3/01*** (2006.01)   ***A61B 5/0476*** (2006.01)
***G06F 17/18*** (2006.01)

(21) Application number: **11782226.2**

(22) Date of filing: **21.10.2011**

(86) International application number:
**PCT/IB2011/054719**

(87) International publication number:
**WO 2013/057544 (25.04.2013 Gazette 2013/17)**

(54) **METHOD OF CALIBRATING AND OPERATING A DIRECT NEURAL INTERFACE SYSTEM**

VERFAHREN ZUM KALIBRIEREN UND BEDIENEN EINES DIREKTEN NEURALEN
SCHNITTSTELLENSYSTEMS

PROCÉDÉ D'ÉTALONNAGE ET DE FONCTIONNEMENT D'UN SYSTÈME D'INTERFACE
NEURONAL DIRECT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.08.2014 Bulletin 2014/35**

(73) Proprietor: **Commissariat à l'Énergie Atomique
et aux Énergies Alternatives
75015 Paris (FR)**

(72) Inventors:
• **STROKOVA AKSENOVA, Tetiana
F-38120 Saint Egreve (FR)**
• **YELISYEYEV, Andriy
Severodonetsk (UA)**

(74) Representative: **Gevers & Orès
41 avenue de Friedland
75008 Paris (FR)**

(56) References cited:
• **ANDREY ELISEYEV ET AL: "Iterative N-way
partial least squares for a binary self-paced
braincomputer interface in freely moving
animals;Iterative N-way PLS for a binary
self-paced BCI in freely moving animals",
JOURNAL OF NEURAL ENGINEERING,
INSTITUTE OF PHYSICS PUBLISHING, BRISTOL,
GB, vol. 8, no. 4, 10 June 2011 (2011-06-10), page
46012, XP020209257, ISSN: 1741-2552, DOI:
10.1088/1741-2560/8/4/046012**

• **ROBERT TIBSHIRANI: "Regression shrinkage
and selection via the lasso: a retrospective",
JOURNAL OF THE ROYAL STATISTICAL
SOCIETY: SERIES B, vol. 73, no. 3, 1 June 2011
(2011-06-01), pages 273-282, XP55040090, ISSN:
1369-7412, DOI:
10.1111/j.1467-9868.2011.00771.x**
• **NAZARPOUR K: "Parallel
Space-Time-Frequency Decomposition of EEG
Signals of Brain Computer Interfacing", 14TH
EUROPEAN SIGNAL PROCESSING
CONFERENCE (EUSIPCO 2006),, 8 September
2006 (2006-09-08), pages 1-4, XP009143718,**
• **ANDREY ELISEYEV ET AL: "Paper;L1-Penalized
N-way PLS for subset of electrodes selection in
BCI experiments;L1-Penalized N-way PLS for
subset of electrodes selection in BCI
experiments", JOURNAL OF NEURAL
ENGINEERING, INSTITUTE OF PHYSICS
PUBLISHING, BRISTOL, GB, vol. 9, no. 4, 25 July
2012 (2012-07-25), page 45010, XP020227766,
ISSN: 1741-2552, DOI:
10.1088/1741-2560/9/4/045010**

**Description**

The invention relates to a method of calibrating and operating a direct neural interface system.

Direct neural interface systems, also known as brain-computer interfaces (BCI) allow using electrophysiological signals issued by the cerebral cortex of a human or animal subject for driving an external device. BCI have been the subject of intense research in the last four decades. At present, a human subject or an animal can drive "by the thought" a simple device, such as a cursor on a computer screen. In 2006, a tetraplegic subject has even been able to drive a robotic arm through a BCI. See the paper by Leigh R. Hochberg et al. "Neuronal ensemble control of prosthetic devices by a human with tetraplegia", Nature 442, 164-171 (13 July 2006).

Until now, the best results in this field have been obtained using invasive systems based on intracortical electrodes. Non-invasive systems using electroencephalographic (EEG) signals have also been tested, but they suffer from the low frequency resolution of these signals. Use of electrocorticographic (ECoG) signals, acquired by intracranial electrodes not penetrating the brain cortex, constitutes a promising intermediate solution. Other kinds of sensors can be used to acquire neural signals, e.g. magnetic field sensors etc.

Conventional BCI systems use a limited number of "features" extracted from EEG or ECoG signals to generate command signals for an external device. These features can be related e.g. to the spectral amplitudes, in a few determined frequency bands, of ECoG signals generated by specific regions of the cortex when the subject imagines performing predetermined action. This approach is not completely satisfactory as, for any different command signal to be generated (e.g. vertical or horizontal movement of a cursor on a screen) it is necessary to identify different features, associated to different actions imagined by the subject and substantially uncorrelated from each other. Especially if the number of different commands signals to be generated is greater than two or three, this can get very complicated. Moreover, this approach is intrinsically inefficient as only a small amount of the information carried by the acquired ECoG signals is exploited.

The paper by K. Nazarpour et al. "Parallel Space-Time-Frequency Decomposition of EEG Signals of Brain Computer Interfacing", Proceedings of the 14th European Signal Processing Conference (EUSIPCO 2006), Florence, Italy, Sept. 4-8, 2006 discloses a method of processing EEG signals, based on multi-way analysis. In the method described by this paper, EEG signals are acquired by 15 electrodes disposed on a subject's scalp. The acquired signals are preprocessed, which includes spatial filtering, digitization and wavelet transform. Preprocessed data are arranged in a three-way tensor, the three ways corresponding to space (i.e. electrode location on the subject's scalp), time and frequency. A tensor corresponding to signals acquired over a 3-second observation window during which the subject imagines moving either the left of the right index is decomposed using the well-known PARAFAC (PARallel FACtors) technique. Then classification is performed using SVM Method (Support Vector Machine), which only enables the classification of observation vectors. Therefore, before the classification step, the tensor corresponding to signals has to be projected on one dimension, namely the spatial dimension. The spatial signatures of the first two PARAFAC factors are fed to a suitable classifier which discriminates between a left index and right index imaginary movement. This method suffers from a few important drawbacks.

First of all, as only the spatial signatures of the PARAFAC factors are used, a large amount of the available information is lost. Furthermore PARAFAC is applied to decompose EEG signal tensor before and independently of classification. Being a generalization of principal component analysis (PCA), PARAFAC projects the tensor to a low dimensional space trying to explain the variability of observations (EEG), keeping the dominant (i.e. most informative) components of signal, but without taking into account their relevance for discrimination. Otherwise stated, non event-related information (useless for discrimination) can be retained, while event-related (and therefore useful for discrimination) components having low amplitude can be lost.

Moreover, a "human" intervention is still required to associate the classifier output to the left or to the right index movement. In other words, this step, the so-called calibration procedure, is not carried out automatically.

Also, only a rather narrow frequency band is considered ($\mu$ band). This band is known to be usable in EEG-based BCI. Otherwise stated, like in "classical" method there is a pre-selection of only a small portion of the available information.

Most prior art BCI systems - including the previously described one by K. Nazarpour et al. - are based on a "cue-paced", or synchronized, approach where subjects are waiting for an external cue that drives interaction. As a result, users are supposed to generate commands only during specific periods. The signals outside the predefined time windows are ignored. However, in a real-life environment this restriction would be very burdensome. As opposed to the "cue-paced" systems, no stimulus is used by "self-paced" BCIs. However, the performances of prior-art self-paced BCIs are not suitable for practical application in particular because of a high level of false system activations, which causes frustration of users and limits the application of the system. Moreover, prior art self-paced BCI experiments were carried out in laboratory conditions, which differ significantly from natural environment where users are not concentrated properly, can be disturbed by external noises, etc. In the majority of prior art self-paced experiments, session time does not exceed several minutes, which is not enough to verify BCI performance. Finally, duration of experiment series is short enough to neglect long-term brain plasticity effects.

The paper by A. Eliseyev et al. "Iterative N-way partial least squares for a binary self-paced brain-computer interface in freely moving animals" discloses a self-paced BCI method based on multi-way analysis, and more precisely on N-way partial least squares (NPLS). This method comprises a calibration step, wherein neuro-electric signals are acquired by a plurality of ECoG electrodes implanted on a rat over a plurality of observation time windows. The acquired signals are conditioned, wavelet-transformed and represented in the form of a 4-way tensor ("observation tensor") X, whose modalities are: observation window / electrode ("space") / time / frequency. Simultaneously, binary data indicative of a voluntary action (pressing a pedal) performed or not by the rat during each of said observation time windows are acquired, and organizing them in a binary vector y ("'output vector"). Then, NPLS regression of **y** on **X** is performed, leading to a regression model. When calibration is completed, the regression model can be used to predict **y** from newly-acquired data **X**. A drawback of NPLS is that it requires very large amounts of memory to store the tensor **X**. For this reasons, the paper proposes a new iterative algorithm, named "INPLS" for "Iterative NPLS" which is based on fragmentation of the dataset into several subsets, and their sequential treatment. Claim 1 is characterized with respect to the disclosure of this paper. The paper further discloses that the claimed weight vectors are chosen such as to maximize the covariance between the claimed score vector and the claimed output vector or tensor. NPLS, PARAFAC and other multi-way statistical methods are described in R. Bro "Multi-way Analysis in the Food Industry - Models, Algorithms, and Applications", PhD Thesis, University of Amsterdam 1998, available on the Internet at URL:http://www.models.kvl.dk/sites/default/files/brothesis_0.pdf

The present invention aims at improving the method described by the paper discussed above, and in particular its calibration step. More precisely, the invention, as defined in claim 1, aims at reducing the prediction error of said method and/or its computational cost.

According to the invention, this aim is achieved by a method of calibrating a direct neural interface system comprising the steps of:

a. Acquiring electrophysiological signals representative of a neuronal activity of a subject's brain over a plurality of observation time windows and representing them in the form of a N+1-way tensor, N being greater or equal to one (preferably, greater or equal to two; in a most preferred embodiment, greater or equal to three), called an observation tensor;

b. Acquiring data indicative of a voluntary action performed by said subject during each of said observation time windows, and organizing them in a vector or tensor, called an output vector or tensor; and

c. Determining a regression function of said output vector or tensor on said observation tensor (multi-way regression when N>1);

wherein said step c. includes performing multilinear decomposition of said observation tensor on a "score" vector, having a dimension equal to the number of said observation time windows, and N "weight" vectors, characterized in that said "weight" vectors are chosen such as to maximize the covariance between said "score" vector and said output vector or tensor subject to a sparsity-promoting constraint or penalty, wherein said "weight" vectors are determined by decomposing a covariance tensor, representing the covariance of said observation tensor and said output vector or tensor, using a penalized Alternating Least Squares algorithm. The introduction of a suitable constraint or penalty term allows obtaining sparse weight vectors, i.e. vectors comprising one or more terms which are exactly zero (or near to zero). This results in a considerable reduction in computational cost, not only during the calibration step, but also during prediction. Moreover, as it will be shown later, the "penalized-NPLS" method of the invention allows outperforming NPLS in terms of prediction error. If N=1, penalized NPLS becomes penalized PLS.

Another object of the invention, as defined by dependent claim 8, is a method of operating a direct neural interface system for interfacing a subject's brain to an external device, said method comprising the steps of:

- acquiring, conditioning, digitizing and preprocessing electrophysiological signals representative of a neuronal activity of said subject's brain over at least one observation time window; and
- generating at least one command signal for said external device by processing said digitized and preprocessed electrophysiological signals;

wherein said step of generating command signals comprises:

- representing the electrophysiological signals acquired over said or each observation time window in the form of a N-way data tensor, N being greater or equal to one (preferably, greater or equal to two; in a most preferred embodiment, greater or equal to three); and
- generating an output signal corresponding to said or each observation time window by performing regression over said or each data tensor (multi-way regression if N>1);

characterized in that said method comprises a calibration step as discussed above.

Particular embodiments of the inventions constitute the subject-matter of the other dependent claims.

Additional features and advantages of the present invention will become apparent from the subsequent description, taken in conjunction with the accompanying drawings, wherein:

- Figure 1 is a functional scheme of a direct neural interface system according to an embodiment of the invention;
- Figures 2 and 3 are schematic illustrations of the signal representation and decomposition used in an embodiment of the invention;
- Figure 4 illustrates the calibration of a direct neural interface system according to an embodiment of the invention; and
- Figures 5 and 6 illustrate the results of an experimental validation of the concept of invention.

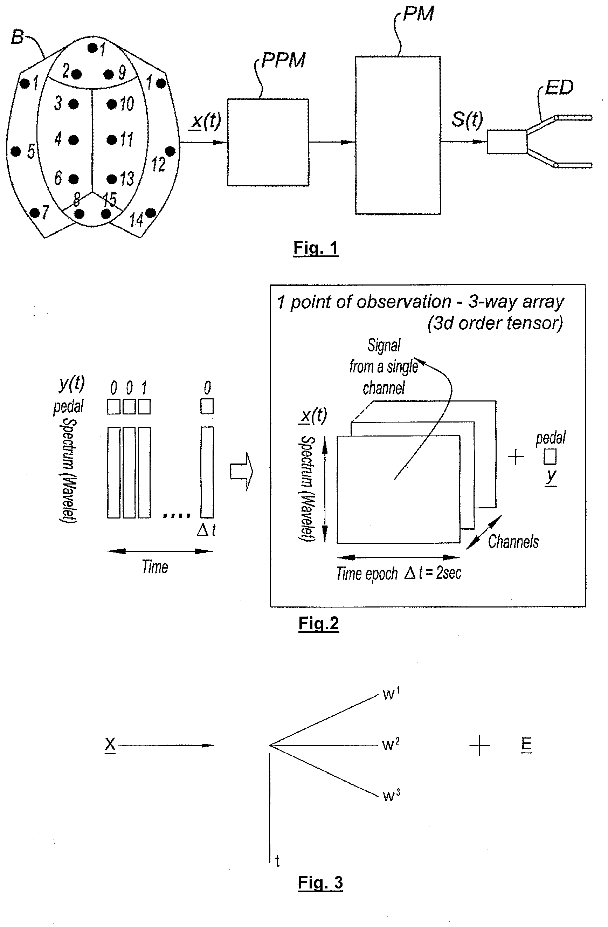

Figure 1 illustrates the general structure of a direct neural interface system according to an exemplary embodiment of the invention. In this embodiment, an intention of a (human or animal) subject to perform a simple action (e.g. press a pedal) is chosen as a specific behavior used for controlling an external device. To collect the data, the brain B of the subject is implanted with fourteen electrodes of measure (references 2 - 15) and three reference electrodes (reference 1). As it is commonly known, the aim of these reference electrodes is to provide a "common signal". By "common signal", it is meant an electrical signal that affects all or most of measurement electrodes. As this signal is less specific to actions, it is usually preferable to evaluate it as precisely as possible, so as to remove it. In this purpose, one or more reference electrodes may be operated. The ECoG signals acquired by the electrodes are pre-processed by pre-processing means PPM, and then processed by processing means PM for generating command signals driving an external device ED (e.g. a manipulator). The pre-processing and processing means can be implemented in the form of application-specific integrated circuits, programmable circuits, microprocessor cards, suitably programmed general-purpose computers, etc.

Pre-processing comprises amplifying and filtering the raw signals acquired by the electrodes, sampling them and converting the sample to digital format. It can also comprise applying a Common Average Reference (CAR) filter:

$$CAR\big(x_i(t)\big) = x_i(t) - \sum\nolimits_{i=1}^{m} x_i(t) / m$$

where $x_i(t)$ is the time-dependent signal acquired by the i-th electrode and m is the number of electrodes of measure (14 in the case of the figure). The application of this common average reference yields to a reduction of a common signal measured by all electrodes.

Processing comprises carrying out a time-frequency analysis of the preprocessed signals over sliding windows, or "epochs" $[t - \Delta\tau, t]$ for all the electrodes. This time-frequency analysis can consist in wavelet decomposition, e.g. based on Mayer wavelets.

As a result, each observation time window (duration $\Delta\tau$, typically of the order of a second, comprising a few tens or hundreds of samples) is associated to a third-order (or "three-way") tensor - or "data cube" - $\underline{\mathbf{x}} \in R^{I_1 \times I_2 \times I_3}$ of independent variables. The dimension $I_1$ corresponds to the number of electrodes; the dimension $I_2$ corresponds to the number of signal samples in an epoch; and the dimension $I_3$ corresponds to the number of frequency bins used in the wavelet decomposition of the signal (e.g. 145 bins, spanning the 10 Hz - 300 Hz band with 2 Hz resolution). Space, time and frequency are also called the "modes" or "modalities" of analysis.

In simplified embodiments of the invention, lower-order tensors could be used. For example, only the temporal and spatial modalities of the signal could be considered, or the frequency and spatial modalities, in which cases $\mathbf{x}$ would be a two-way tensor, i.e. a matrix. A further simplification would consist in using a single electrode and a temporal-only or spectral-only representation of the signal.

Processing also comprises generating command signals S for driving the external device ED by performing multi-way regression over each data tensor $\underline{\mathbf{x}}$ corresponding to an observation time window. The command signal generated for each observation time window can be a Boolean scalar (i.e. a on/off command), an integer scalar (i.e. a discrete, non binary signal), a real scalar (i.e. an analog command), a vector (e.g. for driving a movement of a multi-axis robotic arm) or even a tensor.

The regression equation applied by the processing means to generate the command signal is determined through calibration, which is an important part of the invention.

For calibration, electrophysiological signals are acquired over time windows, or "epochs" $[t-\Delta\tau, t]$, and the corresponding data cubes $\underline{\mathbf{x}}$ are built; simultaneously, a signal y indicative of an action performed by the subject during each epoch is acquired. E.g., in the case of a binary signal, a value of y=1 (respectively: y=0) indicates that the action has been performed (respectively: has not been performed). Variable y is called an "output" or "dependent" variable; actually, it is an input of the calibration algorithm, but it corresponds to the "output variable" of the multi-way regression used for generating the command signal.

A set of "n" observations, each one corresponding to a three-way tensor **x** and an output variable, are used to form a forth order (or four-way) tensor $\underline{\mathbf{X}} \in R^{n \times I_1 \times I_2 \times I_3}$ and an output vector **y** $\in R^n$. This is illustrated on figure 2.

The overall goal of the calibration operation is the regression of the output vector **y** on the tensor of observations $\underline{\mathbf{X}}$.

In the above-referenced paper by A. Eliseyev et al. it has been proposed to perform the regression using multilinear PLS (also known as N-way PLS, or NPLS), because of its efficiency in the case of highly correlated observations. NPLS is a generalization of the widely-known PLS (partial least square technique) to tensor data. The present invention is based on a variant of NPLS, called "penalized NPLS". This technique will be discussed after a brief reminder of "classical" NPLS. For more information on NPLS, the reader can revert by the above-referenced monograph of R. Bro.

Partial Least Squares (PLS) is a statistical method for vector-based analyses of high dimensionality data. PLS properly treats situations when a matrix of observations **X** contains more variables then observations, and said variables are highly correlated. A predictive model is constructed by means of a latent variable t which is derived from **X** in such a way that covariance between **t** and dependent variables vector **y** is maximized. PLS is applied for both regression/classification and for dimensional reduction of the data. As opposed to other widely used projection based methods like Principal Component Analysis (PCA), PLS uses not only independent, but also dependent variables for factorization, which makes it more efficient.

NPLS is a generalization of PLS to the case of tensor independent **X** and/or dependent **Y** variables.

Without loss of generality, only the case of a fourth order observation tensor $\underline{\mathbf{X}} \in R^{n \times I_1 \times I_2 \times I_3}$ and a vector **y** $\in R^n$ is considered in detail. Generalization is straightforward. Both **X** and y are centered along the first dimension, i.e. their mean value in time is set equal to zero.

NPLS models tensor $\underline{\mathbf{X}}$ by means of a "latent variable" **t** $\in R^n$ extracted from the first mode of **X** in such way that covariance between *t* and $\bar{y}$ is maximized. In addition to vector *t*, the algorithm forms a set of "weight" or "loading" vectors $\{w^1 \in R^{I_1}, w^2 \in R^{I_2}, w^3 \in R^{I_3}\}$ related to the second, the third, and the forth modality of **X**, respectively.

The first step of NPLS consists in decomposing $\underline{\mathbf{X}}$ into a "score" vector **t** $\in R^n$ and a set of "weight" (or "loading") vectors $\mathbf{w}^k \in R^{I_k}$, k = 1,2,3 :

$$x_{j,i_1,i_2,i_3} = t_j w_{i_1}^1 w_{i_2}^2 w_{i_3}^3 + e_{j,i_1,i_2,i_3} . \tag{1}$$

In tensor notation

$$\underline{\mathbf{X}} = \mathbf{t} \circ (\mathbf{w}^1 \circ \mathbf{w}^2 \circ \mathbf{w}^3) + \underline{\mathbf{E}} \tag{1'}$$

where $\circ$ is the tensors product. Decomposition is generally not exact; this is accounted for by the residual tensor $\underline{\mathbf{E}}$. This decomposition is illustrated schematically on figure 3.

Each weight $\mathbf{w}^k$ corresponds to a mode of analysis: $\mathbf{w}^1$ represents a time signature, $\mathbf{w}^1$ represents a spectral signature ad $\mathbf{w}^3$ represents a spatial signature.

For given set of $\mathbf{w}^k$

$$t_j = \sum_{i_1,i_2,i_3} x_{j,i_1,i_2,i_3} w_{i_1}^1 w_{i_2}^2 w_{i_3}^3 \tag{2}$$

provides the least squares solution for (1) under the constrains $\|\mathbf{w}^1\| = \|\mathbf{w}^3\| = \|\mathbf{w}^3\| = 1$.

In conventional NPLS, the weights $\mathbf{w}^k$ are chosen in order to maximize the covariance **t** and **y**. It can be formalized as the following optimization problem:

$$\left\{ \mathbf{w}^1, \mathbf{w}^2, \mathbf{w}^3 \right\} = \arg\min \left( \left\| \underline{\mathbf{Z}} - \mathbf{w}^1 \circ \mathbf{w}^2 \circ \mathbf{w}^3 \right\|_F \right) \tag{3}$$

where:

- $\|\cdot\|_F$ is the Frobenius norm;
- "$\circ$" is the tensors product;
- Tensor $\underline{\mathbf{Z}} = \underline{\mathbf{X}} \times_1 \mathbf{y}$, where "$\times_1$" is the first-modality vector product, represents the covariance of $\underline{\mathbf{X}}$ and **y**.

The decomposition (3) can be computed using e.g. PARAFAC or the Alternating Least Squares algorithm.

A coefficient $b_1$ of a regression $\mathbf{y}=b_1\mathbf{t}+\mathbf{f}_1$ is then calculated using least squares.

Residual $\underline{\mathbf{E}}$ can also be decomposed, resulting in a second set of "score" and "weight" vectors, and so on. Each of these sets is called a "factor" of the decomposition. This iterative procedure is known as deflation.

At each deflation step, new values of dependent and independent variables are given by:

$$\underline{\mathbf{X}}_{new} = \underline{\mathbf{X}} - \mathbf{t} \circ \mathbf{w}^1 \circ \mathbf{w}^2 \circ \mathbf{w}^3 \ (\text{i.e. } \underline{\mathbf{X}}_{new} = \underline{\mathbf{E}}) \qquad (4a)$$

$$\mathbf{y}_{new} = \mathbf{y} - \mathbf{T}\mathbf{b} \qquad (4b)$$

where matrix $\mathbf{T} = [\mathbf{t}_1|...|\mathbf{t}_f]$ is composed from all score vectors obtained on the previous $f$ steps, $\mathbf{b}$ is defined as: $\mathbf{b} = (\mathbf{T}^T\mathbf{T})^{-1}\mathbf{T}^T\mathbf{y}$ ("T" exponent means transposition, "-1" exponent means inversion). Residuals $\underline{\mathbf{X}}_{new}$ and $\mathbf{y}_{new}$ are used to find the next set of weights and score (loading) vectors.

In other words, $\underline{\mathbf{X}}_{f+1} = \underline{\mathbf{X}}_f - \mathbf{t}_f \circ \mathbf{w}_f^1 \circ \mathbf{w}_f^2 \circ \mathbf{w}_f^3$
and $\mathbf{y}_{f+1} = \mathbf{y}_f - \mathbf{T}_f\mathbf{b}_f$
The index $f$ indicating the iteration rank ($1 \leq f \leq F$).

Equation (4b) provides a linear relation between output variable $\mathbf{y}$ and latent variables ($\mathbf{T}$). A non linear equation might be applied as well.

After $F$ steps, the regression equation becomes:

$$\hat{\mathbf{y}} = \mathbf{t}_1 b_1 + \left[\mathbf{t}_1\mathbf{t}_2\right]\mathbf{b}_2 + ... + \left[\mathbf{t}_1\mathbf{t}_2...\mathbf{t}_F\right]\mathbf{b}_F \qquad (5)$$

which can be rewritten more compactly as:

$$\hat{\mathbf{y}} = \mathbf{T}\mathbf{b} \qquad (6)$$

It is to be noticed that the dimension of each vector $b_f$ is $f$.

The latent variable can also be normalized: $\mathbf{t}^*_f = \mathbf{t}_f/\|\mathbf{t}_f\|$, in which case the regression equation, or "predictive model", becomes:

$$\hat{\mathbf{y}}^* = \mathbf{T}^*\mathbf{b}^* \qquad (6')$$

with $b^*_f = t_f\|\mathbf{t}_f\|$

Calibration yields $\mathbf{b}$ and $\{\mathbf{w}_f^1, \mathbf{w}_f^1, \mathbf{w}_f^1\}$ for f=1 ... F. During operation of the BCI system, input data $\underline{\mathbf{X}}$ are decomposed using the weight $\{\mathbf{w}_f^1, \mathbf{w}_f^1, \mathbf{w}_f^1\}$ to give $\mathbf{T}$, then $\hat{\mathbf{y}}$ is computed by applying (6) and used to determine the command signal S.

During the prediction step the input data consist of a single data cube $\underline{\mathbf{x}}$, and T is a transposed vector of latent variables.

Penalized NPLS differs from conventional PLS by the use of a different decomposition of the tensor $\underline{\mathbf{Z}}$, yielding weight vectors $\{\tilde{\mathbf{z}}_1, \tilde{\mathbf{z}}_2, \tilde{\mathbf{z}}_3\}$ :

$$\{\tilde{\mathbf{z}}_1, \tilde{\mathbf{z}}_2, \tilde{\mathbf{z}}_3\} = \arg\min\left(\|\underline{\mathbf{Z}} - \mathbf{z}_1 \circ \mathbf{z}_2 \circ \mathbf{z}_3\|^2_F + P(\mathbf{z}_1, \mathbf{z}_2, \mathbf{z}_3)\right) \qquad (7)$$

where $P(\cdot)$ is a penalization term chosen to promote sparsity of the weight vectors. It is known that e.g. penalty terms based on a L1-norm (or, equivalently, L1-constraints) have the property of promoting the sparsity of the solution of a quadratic optimization problem. Other sparsity-promoting penalizations are known and can be used to carry out the invention, e.g. all $L_p$ norm penalties with $0 \leq p \leq 1$.

As it is known in the art, equation (7) is equivalent to a constrained optimization problem.

Suitable penalty functions / constraints are e.g. the so-called:

- LASSO (Least Absolute Shrinkage Selection Operator): $P(A)=\|A\|_1$ - see R. Tibshirani "Regression shrinkage and variable selection via lasso", J. Roy. Statistic. Soc. Ser. B 58, 267-288 (1996);
- Fused LASSO: $P(A)=\|DA\|_1$, where D is a difference operator - see R. Tibshirani et al. "Sparsity and smoothness via the fused lasso », R. Statist. Soc. B (2005) 67, Part 1, pp. 91-108;

- Elastic Net, which combines L1- and L2-norm penalization terms - see H. Zou, T. Hastie "Regularization and variable selection via the elastic net" J. Roy. Statistic. Soc. Ser. B 67, 301-320.

In the following, the special case of a L1 penalization term will be considered: $P(\mathbf{z_1},\mathbf{z_2},\mathbf{z_3}) = \lambda_1\|\mathbf{z_1}\|_1 + \lambda_2\|\mathbf{z_2}\|_1 + \lambda_3\|\mathbf{z_3}\|_1$, where $\lambda_1$, $\lambda_2$ and $\lambda_3$ are real penalization parameters. The decomposition of tensor $\underline{Z}$ according to equation (7) becomes then:

$$\{\widetilde{\mathbf{z}}_1,\widetilde{\mathbf{z}}_2,\widetilde{\mathbf{z}}_3\} = \arg\min\left(\left\|\underline{Z} - \mathbf{z_1}\circ\mathbf{z_2}\circ\mathbf{z_3}\right\|_F^2 + \lambda_1\|\mathbf{z_1}\|_1 + \lambda_2\|\mathbf{z_2}\|_1 + \lambda_3\|\mathbf{z_3}\|_1\right) \qquad (7')$$

For the sake of simplicity, in an exemplary embodiment of the invention penalization will only be applied to the first weight vector, corresponding to the "spatial" modality of tensor $\underline{Z}$: $\lambda_1 = \lambda$; $\lambda_2 = \lambda_3 = 0$. Equation (7) becomes then:

$$\{\widetilde{\mathbf{z}}_1,\widetilde{\mathbf{z}}_2,\widetilde{\mathbf{z}}_3\} = \arg\min\left(\left\|\underline{Z} - \mathbf{z_1}\circ\mathbf{z_2}\circ\mathbf{z_3}\right\|_F^2 + \lambda\|\mathbf{z_1}\|_1\right) \qquad (7'')$$

One of the way to solve the problem is the Alternative Least Squares algorithm, which is an iterative procedure:

*1st step:*

$z_1$, $z_2$ and $z_3$ are fixed to initial values, with all terms being 1.

*$i^{th}$ step:*

$z_2$ and $z_3$ are fixed and $z_1$ is determined as follows:

$$\widetilde{z}_1 = \arg\min_{z_1}\left(\left\|\underline{Z} - \mathbf{z_1}\circ\mathbf{z_2}\circ\mathbf{z_3}\right\|_F^2 + \lambda\|\mathbf{z_1}\|_1\right);$$

$z_1$ and $z_3$ are fixed and $z_2$ is determined as follows:

$$\widetilde{z}_2 = \arg\min_{z_2}\left(\left\|\underline{Z} - \mathbf{z_1}\circ\mathbf{z_2}\circ\mathbf{z_3}\right\|_F^2 + \lambda\|\mathbf{z_1}\|_1\right) = \arg\min_{z_2}\left(\left\|\underline{Z} - \mathbf{z_1}\circ\mathbf{z_2}\circ\mathbf{z_3}\right\|_F^2\right)$$

Since $\|\mathbf{z_1}\|_1$ is constant at this step:

$$\widetilde{z}_2 = \arg\min_{z_2}\left(\left\|\underline{Z} - \mathbf{z_1}\circ\mathbf{z_2}\circ\mathbf{z_3}\right\|_F^2\right)$$

$z_1$ and $z_2$ are fixed and $z_3$ is determined as follows:

$$\widetilde{z}_3 = \arg\min_{z_3}\left(\left\|\underline{Z} - \mathbf{z_1}\circ\mathbf{z_2}\circ\mathbf{z_3}\right\|_F^2 + \lambda\|\mathbf{z_1}\|_1\right) = \arg\min_{z_3}\left(\left\|\underline{Z} - \mathbf{z_1}\circ\mathbf{z_2}\circ\mathbf{z_3}\right\|_F^2\right)$$

Until convergence of $\{\widetilde{\mathbf{z}}_1,\widetilde{\mathbf{z}}_2,\widetilde{\mathbf{z}}_3\}$

The three optimization problems to be solved during each step can be written in matrix form

$$\widetilde{\mathbf{z}}_1 = \arg\min_{\mathbf{z_1}}\left(\left\|\mathbf{Z}_{(1)} - \mathbf{z_1}\mathbf{z}_{2,3}^T\right\|_F^2 + \lambda\|\mathbf{z_1}\|_1\right), \qquad (8A)$$

$$\widetilde{\mathbf{z}}_2 = \arg\min_{\mathbf{z}_2}\left(\left\|\mathbf{Z}_{(2)} - \mathbf{z}_2\mathbf{z}_{1,3}^T\right\|_F^2\right) \qquad (8B)$$

$$\widetilde{\mathbf{z}}_3 = \arg\min_{\mathbf{z}_3}\left(\left\|\mathbf{Z}_{(3)} - \mathbf{z}_3\mathbf{z}_{1,2}^T\right\|_F^2\right). \qquad (8C)$$

where $\mathbf{Z}_{(1)}$ is the matrix resulting from the unfolding of the tensor Z along the first modality: $\mathbf{Z}_{(1)} = \underline{\mathbf{Z}}_{(1)}$, $\mathbf{z}_{2,3}$ = vect($\mathbf{z}_2 \circ \mathbf{z}_3$) and so on.

Optimization problems (8B, 8C) have an analytical solution:

$$\widetilde{\mathbf{z}}_2 = \mathbf{Z}_{(2)}\mathbf{z}_{1,3}\left(\mathbf{z}_{1,3}^T\mathbf{z}_{1,3}\right)^{-1} \qquad (9A)$$

$$\widetilde{\mathbf{z}}_3 = \mathbf{Z}_{(3)}\mathbf{z}_{1,2}\left(\mathbf{z}_{1,2}^T\mathbf{z}_{1,2}\right)^{-1} \qquad (9B)$$

To solve optimization problem (8A) numerical optimization is applied. For example Gauss-Seidel algorithm can be applied - see e.g. M. Schmidt. "Least Squares Optimization with L1-Norm Regularization", Cs542B Project Report, December 2005; available on the internet at URL:http://www.di.ens.fr/~mschmidt/Software/lasso.pdf.

When convergence is reached, the weight vectors $w_1$, $w_2$ and $w_3$ are taken equal to the $\{\widetilde{\mathbf{z}}_1,\widetilde{\mathbf{z}}_2,\widetilde{\mathbf{z}}_3\}$ : $\{\mathbf{w}^1,\mathbf{w}^2,\mathbf{w}^3\}$ = $\{\widetilde{\mathbf{z}}_1,\widetilde{\mathbf{z}}_2,\widetilde{\mathbf{z}}_3\}$.

Vectors $\mathbf{w}^1,\mathbf{w}^2,\mathbf{w}^3$ represent projectors for each modality. Elements of $\mathbf{w}^1$ are projection coefficients for every electrode. Elements of $\mathbf{w}^2$ are projection coefficients for every frequency and so on.

If the value of the $\lambda$ parameter is sufficiently high, at the end of the iterative optimization many - or even most - of the component of $\mathbf{z}_1$ (and therefore $\mathbf{w}^1$) will be equal or near to zero, i.e. $\mathbf{z}_1$ ($\mathbf{w}^1$) will be a sparse vector, which is desirable. A too low value of $\lambda$ would fail in achieving sparsity, while a too high value could result in setting all the components of $\mathbf{z}_1$ to zero. More precisely, if , $\lambda \geq \lambda_{max} = \max\left(2\mathbf{z}_{2,3}^T\underline{\mathbf{Z}}_{(1)}^T\right)$ Gauss-Seidel algorithm will return as a solution $\widetilde{\mathbf{z}}_1 = \mathbf{0}$.

Therefore, the setting of $\lambda$ (or, more generally, of $\lambda_1$, $\lambda_2$ and $\lambda_3$) is an important part of the method. This setting can be performed manually, by trials and errors, or using an automatic approach such as:

- cross-validation, see R. Kohavi, "A study of cross-validation and bootstrap for accuracy estimation and model selection". Proceedings of the Fourteenth International Joint Conference on Artificial Intelligence 2 (12): 1137-1143 (1995).
- generalized cross-validation, see G. Golub et al."Generalized cross-validation as a method for choosing a good ridge parameter", Technometrics, 21, 215 - 223 (1979);
- Akaike's Information Criterion, see H. Akaike, "A new look at the statistical model identification" IEEE Trans. Automat. Control 19, 716 - 723 (1974); or
- Schwartz's Bayesian Information Criterion, see G. Schwartz "Estimating the dimension of a model", Ann. Statist. 6, 461-464 (1978).

The criterion or cross-validation procedure is applied to the final regression model.

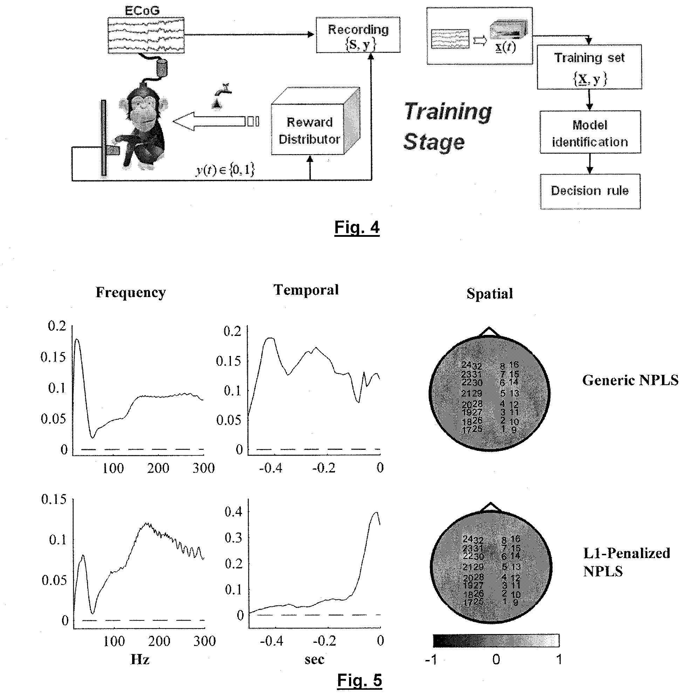

The invention has been subject to experimental validation, as illustrated on figure 4.

Data was collected from behavioral experiments in non-human primates (monkeys) based on a simple reward-oriented task. During the experiment the monkey is sitting in a custom made primate chair minimally restrained, its neck collar hooked to the chair. The monkey has to push a pedal which can be mounted in for different positions ("left", "right", "up", and "down") on a vertical panel facing the monkey. Every correct push event activates a food dispenser. No cue or conditioning stimulus was used to tell the monkey when to push the pedal. A set of ECoG recordings was collected from 32 surface electrodes chronically implanted in the monkey's brain. Simultaneously, information about the state of the pedal was stored. One recording of each position was used to calibrate the BCI system. Training data sets included all event-related epochs and randomly selected "non-event" epochs.

To calibrate the BCI system the brain activity signal of the training recording was mapped to the temporal-frequency-spatial space to form a tensor of observation. For each epoch $j$ (determined by its final time $t$), electrode $c$, frequency $f$ and time shift $\tau$, elements $x_{j,\tau,f,c}$ of the tensor $\underline{\mathbf{x}}$ were calculated as norm of the continuous wavelet transform of ECoG signal (see Fig. 1). Frequency band [10,300] Hz with step $\delta f$ = 2 Hz and sliding windows [$t - \Delta\tau, t$], $\Delta\tau$ = 0.5 s with step $\delta\tau$ = 0.01 s were considered for all electrodes $c = \overline{1,32}$. The resulting dimension of a data cube $\underline{\mathbf{x}}$ is (146×51×32). Meyer wavelet was chosen as the mother wavelet taking into account its computational efficiency. The binary dependent variable was set to one - $y_j$ = 1 - if the pedal was pressed at time $t$, and $y_j$ = 0, otherwise.

The resulting tensor and the binary vector, indicating the pedal position, were used for calibration. Five factors (the number was defined using cross-validation procedure) and the corresponding latent variables $t_i, i = \overline{1,5}$ were extracted by the L1-penalized version of the NPLS algorithm ($\lambda_1 = \lambda = 0.9\lambda_{max}$).

Modality Influence (MI) analysis was applied to estimate the relative importance of the elements of each mode for the final predictive model, i.e. the relative importance of the electrodes, of the frequency bands and of the time intervals related to the control events. For an introduction to MI Analysis, see R.D. Cook and S. Weisberg "Residuals and Influence in Regression", Chapman and Hall, 1982.

The elements of input data participate in the NPLS regression model implicitly, through the latent variables. Modality Influence (MI) analysis allows estimating relative importance of the elements of each mode for the final model. We applied MI to estimate the importance of electrodes, frequencies bands, and time intervals related to control events.

In case of tensor input and scalar output variables, the MI procedure is as follows. Latent variables are normalized

$\mathbf{t}_f^* = \mathbf{t}_f / \left\| \mathbf{t}_f \right\|$ and the regression model takes the form: $\hat{\mathbf{y}} = \mathbf{T}^*\mathbf{b}^*$, $b_f^* = b_f \left\| \mathbf{t}_f \right\|$ $f = \overline{1,F}$. Then, for the chosen modality

$i$ = 1,2,3, the coefficients $\mathbf{b}^*$ and the components of all the factors related to this modality $\left\{ \mathbf{w}_f^i \right\}_{f=1}^F$ are used to form the

matrix $\mathbf{A}^i = \left[ b_1^* \mathbf{w}_1^i \mid \ldots \mid b_F^* \mathbf{w}_F^i \right]$. The vector of leverages $\mathbf{h}^i = diag(\mathbf{A}^i(\mathbf{A}^i)^T)$ shows the summarized influence of elements of this modality on the predicted output.

The results of MI analysis applied to the exemplary BCI system discussed here are illustrated on the bottom line of figure 5. The top line corresponds to the results obtained using non-penalized ("generic") NPLS. Time and frequency modalities are represented by plots, spatial modality by grayscale maps. It can easily be seen that use of L1-penalized NPLS leads to a sparse contribution of electrodes: only a few electrodes have an influence different from zero, and electrode n°22 largely dominates. On the contrary, in the case of "generic" NPLS all the electrodes have similar contributions. L1-penalization has only been applied to the spatial modality, therefore the frequency and temporal modalities are not sparse. More precisely, Modality Influence analysis indicates that the electrode n°22 located in the primary motor cortex has the highest impact on the decision rule (84%, 97%, 89%, and 75% of extracted information for "left", "right", "up", and "down" positions of the pedal, respectively). High frequencies (≥100 Hz) significantly contribute to the decision in the frequency modality, however, the influence of the lower frequencies (less than 100 Hz) is also considerable, especially for the "left" position of the pedal. In the time domain the interval [-0.2, 0] s before the event is the most significant for all positions of the pedal.

The sparsity of the spatial modality allowed using small subsets of electrodes for building the predictive models: 6 electrodes were used for "left" and "right", 7 for "up" and 9 for "down". It is clear that this greatly reduces the computational complexity of the algorithm; for instance, signals coming from 13 to 16 of the 22 electrodes do not contribute to prediction and therefore they do not even have to be processed.

The resulting coefficients $b_i^*$, of the normalized predictive model $\hat{y} = \sum_{i=1}^5 t_i^* b_i^*$, corresponding to the weights of the related factors in the final decomposition, were:

"left": 0.346, 0.273, 0.232, 0.111, 0.038;
"right": 0.346, 0.217, 0.195, 0.138, 0.104;
"up": 0.383, 0.263, 0.158, 0.151, 0.045;
"down": 0.278, 0.210, 0.194, 0.182, 0.138.

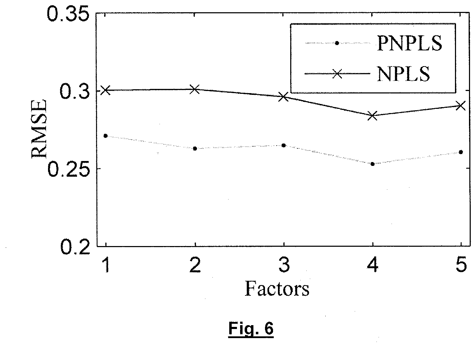

Figure 6 shows the root mean square prediction error (RMSE) for the "up" position of the pedal, corresponding to the "general" NPLS and the "penalized" NPLS (PNPLS) algorithms for different number of factors, comprised between 1 and 5. It can be seen that the L1-Penalized NPLS outperformed the generic NPLS approach for all tested number of factors from 1 to 5. Otherwise stated, besides reducing the computational complexity, PNLS leads to better results than "generic" NPLS.

**Claims**

1. A method of calibrating a direct neural interface system comprising the steps of:

   a. Acquiring electrophysiological signals representative of a neuronal activity of a subject's brain over a plurality of observation time windows and representing them in the form of a N+1-way tensor (X), N being greater or equal to one, called an observation tensor;
   b. Acquiring data indicative of a voluntary action performed by said subject during each of said observation time windows, and organizing them in a vector or tensor (**y**), called an output vector or tensor; and
   c. Determining a regression function of said output vector or tensor on said observation tensor;

   wherein said step c. includes performing multilinear decomposition of said observation tensor on a score vector (**t**), having a dimension equal to the number of said observation time windows, and N weight vectors (**w$^1$, w$^2$, w$^3$**), **characterized in that** said weight vectors are chosen such as to maximize the covariance between said score vector and said output vector or tensor subject to a sparsity-promoting constraint or penalty, wherein said weight vectors are determined by decomposing a covariance tensor, representing the covariance of said observation tensor and said output vector or tensor, using a penalized Alternating Least Squares algorithm.

2. A method according to claim 1, wherein said sparsity-promoting constraint or penalty is based on an L1-norm of said weight vectors.

3. A method according to claim 2, wherein said sparsity-promoting constraint or penalty is based on a penalty operator chosen among: Least Absolute Shrinkage and Selection Operator (LASSO), fused LASSO and Elastic Net.

4. A method according to any of the preceding claims, wherein a Gauss-Seidel algorithm is used to carry out said Alternating Least Squares algorithm.

5. A method according to any of the preceding claims, comprising automatically determining at least one penalization parameter of said sparsity-promoting penalization.

6. A method according to any of the preceding claims, wherein said electrophysiological signals are acquired using a plurality of sensors (1 - 15) associated to different regions of said subject's brain, and subject to time-frequency analysis, and wherein said observation tensor is a four-way data tensor comprising:

   - a first modality, corresponding to said observation time windows;
   - a second modality, corresponding to the sensors used to acquire said electrophysiological signals;
   - a third modality, corresponding to a temporal dimension of a time-frequency representation of said electrophysiological signals; and
   - a fourth modality, corresponding to a frequency dimension of a time-frequency representation of said electrophysiological signals.

7. A method according to claim 6, wherein said sparsity-promoting constraint or penalty acts at least on said second modality resulting in a selection of a subset of said sensors.

8. A method of operating a direct neural interface system for interfacing a subject's brain (B) to an external device (ED), said method comprising the steps of:

   - acquiring, conditioning, digitizing and preprocessing electrophysiological signals representative of a neuronal activity of said subject's brain over at least one observation time window; and
   - generating at least one command signal for said external device by processing said digitized and preprocessed electrophysiological signals;

   wherein said step of generating command signals comprises:

   - representing the electrophysiological signals acquired over said at least one observation time window in the form of at least one N-way data tensor, N being greater or equal to one; and
   - generating an output signal corresponding to said at least one observation time window by performing regression over said at least one data tensor;

**characterized in that** said method comprises a calibration step according to any of claims 1 to 7.

9. A method according to claim 8, wherein the generation of said at least one command signal is self-paced.

10. A method according to any of claims 8 or 9, comprising performing penalized partial least squares regression or multi-way partial least square regression, over said N-way data tensor.

11. A method according to any of the preceding claims, wherein said electrophysiological signals are electrocorticographic (ECoG) signals.


**Patentansprüche**

1. Verfahren zum Kalibrieren eines direkten neuralen Schnittstellensystems, umfassend die Schritte:

   a. Erfassen von elektrophysiologischen Signalen, die für eine neuronale Aktivität des Gehirns einer Person repräsentativ sind, über eine Vielzahl von Beobachtungszeitfenstern und Darstellen derselben in Form eines N+1-Weg-Tensors ($\underline{\mathbf{X}}$), wobei N größer oder gleich eins ist und als Beobachtungstensor bezeichnet wird;
   b. Erfassen von Daten, die eine von der Person während jedes der Beobachtungszeitfenster durchgeführte freiwillige Aktion anzeigen, und Organisieren dieser Daten in einem Vektor oder Tensor ($\mathbf{y}$), der Ausgangsvektor oder Tensor genannt wird; und
   c. Bestimmen einer Regressionsfunktion des Ausgangsvektors oder Tensors auf dem Beobachtungstensor;

   wobei der Schritt c. das Durchführen einer multilinearen Zerlegung des Beobachtungstensors an einem Bewertungsvektor ($\mathbf{t}$), der eine Dimension aufweist, die gleich der Anzahl der Beobachtungszeitfenster ist, und N Gewichtsvektoren ($\mathbf{w}^1$, $\mathbf{w}^2$, $\mathbf{w}^3$) einschließt, **dadurch gekennzeichnet, dass** die Gewichtungsvektoren so gewählt werden, dass die Kovarianz zwischen dem Bewertungsvektor und dem Ausgangsvektor oder Tensor, der einer seltenheitsfördernden Einschränkung oder Strafe unterliegt, maximiert wird, wobei die Gewichtsvektoren durch Zerlegen eines Kovarianztensors, der die Kovarianz des Beobachtungstensors und des Ausgangsvektors oder Tensors darstellt, unter Verwendung eines bestraften alternierenden Algorithmus der kleinsten Quadrate bestimmt werden.

2. Verfahren nach Anspruch 1, wobei die seltenheitsfördernde Einschränkung oder Strafe auf einer L1-Norm der Gewichtungsvektoren basiert.

3. Verfahren nach Anspruch 2, wobei die seltenheitsfördernde Beschränkung oder Strafe auf einem Strafoperator basiert, der ausgewählt ist unter: geringste absolute Schrumpfung und Auswahloperator (LASSO), fusionierter LASSO und elastisches Net.

4. Verfahren nach einem der vorstehenden Ansprüche, wobei ein Gauß-Seidel-Algorithmus verwendet wird, um den alternierenden Algorithmus der kleinsten Quadrate auszuführen.

5. Verfahren nach einem der vorstehenden Ansprüche, umfassend das automatische Bestimmen von mindestens einem Bestrafungsparameter der seltenheitsfördernden Bestrafung.

6. Verfahren nach einem der vorstehenden Ansprüche, wobei die elektrophysiologischen Signale unter Verwendung einer Vielzahl von Sensoren (1-15) erfasst werden, die verschiedenen Bereichen des Gehirns der Person zugeordnet sind und einer Zeit-Frequenz-Analyse unterzogen werden und wobei der Beobachtungstensor ein Vierwege-Datentensor ist, umfassend:

   - eine erste Modalität, die den Beobachtungszeitfenstern entspricht;
   - eine zweite Modalität, die den Sensoren entspricht, die zum Erfassen der elektrophysiologischen Signale verwendet werden;
   - eine dritte Modalität, die einer zeitlichen Dimension einer Zeit-Frequenz-Darstellung der elektrophysiologischen Signale entspricht; und
   - eine vierte Modalität, die einer Frequenzdimension einer Zeit-Frequenz-Darstellung der elektrophysiologischen Signale entspricht.

**7.** Verfahren nach Anspruch 6, wobei die seltenheitsfördernde Beschränkung oder Strafe zumindest auf die zweite Modalität einwirkt, was zu einer Auswahl einer Teilmenge der Sensoren führt.

**8.** Verfahren zum Betrieb eines direkten neuralen Schnittstellensystems zum Verbinden des Gehirns (B) einer Person mit einer externen Vorrichtung (ED), wobei das Verfahren die folgenden Schritte umfasst:

- Erfassen, Konditionieren, Digitalisieren und Vorverarbeiten von elektrophysiologischen Signalen, die für eine neuronale Aktivität des Gehirns der Person repräsentativ sind, über mindestens ein Beobachtungszeitfenster; und
- Erzeugen mindestens eines Befehlssignals für die externe Vorrichtung durch Verarbeiten der digitalisierten und vorverarbeiteten elektrophysiologischen Signale;

wobei der Schritt des Erzeugens von Befehlssignalen umfasst:

- Darstellen der elektrophysiologischen Signale, die über das mindestens eine Beobachtungszeitfenster in Form mindestens eines N-Wege-Datentensors erfasst wurden, wobei N größer oder gleich Eins ist; und
- Erzeugen eines Ausgangssignals, das dem mindestens einen Beobachtungszeitfenster entspricht, durch Ausführen einer Regression über den mindestens einen Datentensor;

**dadurch gekennzeichnet, dass** das Verfahren einen Kalibrierungsschritt nach einem der Ansprüche 1 bis 7 umfasst.

**9.** Verfahren nach Anspruch 8, wobei die Erzeugung des mindestens einen Befehlssignals selbst bestimmt ist.

**10.** Verfahren nach einem der Ansprüche 8 oder 9, umfassend das Durchführen einer bestraften partiellen Regression der kleinsten Quadrate oder einer partiellen Mehrwege-Regression der kleinsten Quadrate über den N-Wege-Datentensor.

**11.** Verfahren nach einem der vorstehenden Ansprüche, wobei die elektrophysiologischen Signale elektrokortikografische (ECoG) Signale sind.

**Revendications**

**1.** Procédé d'étalonnage d'un système d'interface neuronal direct comprenant les étapes de :

a. acquisition de signaux électro-physiologiques représentatifs d'une activité neuronale du cerveau d'un sujet sur une pluralité de fenêtres de temps d'observation et de représentation de ceux-ci sous la forme d'un tenseur à N+1 voies ($\underline{X}$), N étant supérieur ou égal à un, appelé tenseur d'observation ;
b. acquisition de données indicatives d'une action volontaire réalisée par ledit sujet durant chacune desdites fenêtres de temps d'observation, et d'organisation de celles-ci dans un vecteur ou tenseur ($y$), appelé vecteur ou tenseur de sortie ; et
c. détermination d'une fonction de régression dudit vecteur ou tenseur de sortie sur ledit tenseur d'observation ;

dans lequel ladite étape c. inclut la réalisation d'une décomposition multilinéaire dudit tenseur d'observation sur un vecteur de marque ($t$), ayant une dimension égale au nombre de dites fenêtres de temps d'observation, et N vecteurs de poids ($w^1$, $w^2$, $w^3$), **caractérisé en ce que** lesdits vecteurs de poids sont choisis de manière à maximiser la covariance entre ledit vecteur de marque et ledit vecteur ou tenseur de sortie soumis à une contrainte ou pénalité favorisant la parcimonie, dans lequel lesdits vecteurs de poids sont déterminés en décomposant un tenseur de covariance, représentant la covariance dudit tenseur d'observation et dudit vecteur ou tenseur de sortie, en utilisant un algorithme des Moindres Carrés Alternés pénalisés.

**2.** Procédé selon la revendication 1, dans lequel ladite contrainte ou pénalité favorisant la parcimonie est basée sur une norme L1 desdits vecteurs de poids.

**3.** Procédé selon la revendication 2, dans lequel ladite contrainte ou pénalité favorisant la parcimonie est basée sur un opérateur de pénalité choisi parmi : LASSO (Least Absolute Shrinkage and Selection Operator), LASSO fusionné et Filet Élastique.

**4.** Procédé selon l'une quelconque des revendications précédentes, dans lequel un algorithme de Gauss-Seidel est utilisé pour réaliser ledit algorithme des Moindres Carrés Alternés.

**5.** Procédé selon l'une quelconque des revendications précédentes, comprenant la détermination automatique d'au moins un paramètre de pénalisation de ladite pénalisation favorisant la parcimonie.

**6.** Procédé selon l'une quelconque des revendications précédentes, dans lequel lesdits signaux électro-physiologiques sont acquis en utilisant une pluralité de capteurs (1-15) associés à différentes régions du cerveau dudit sujet, et soumis à une analyse de temps-fréquence, et

dans lequel ledit tenseur d'observation est un tenseur de données à quatre voies comprenant :

- une première modalité, correspondant auxdites fenêtres de temps d'observation ;
- une deuxième modalité, correspondant aux capteurs utilisés pour acquérir lesdits signaux électro-physiologiques ;
- une troisième modalité, correspondant à une dimension temporale d'une représentation temps-fréquence desdits signaux électro-physiologiques ; et
- une quatrième modalité, correspondant à une dimension fréquentielle d'une représentation temps-fréquence desdits signaux électro-physiologiques.

**7.** Procédé selon la revendication 6, dans lequel ladite contrainte ou pénalité favorisant la parcimonie agit au moins sur ladite deuxième modalité résultant dans une sélection d'un sous-ensemble desdits capteurs.

**8.** Procédé de fonctionnement d'un système d'interface neuronal direct pour interfacer le cerveau (B) d'un sujet avec un dispositif externe (ED), ledit procédé comprenant les étapes de :

- acquisition, conditionnement, numérisation et prétraitement de signaux électro-physiologiques représentatifs d'une activité neuronale du cerveau dudit sujet sur au moins une fenêtre de temps d'observation ; et
- génération d'au moins un signal d'instruction pour ledit dispositif externe en traitant lesdits signaux électro-physiologiques numérisés et prétraités ;

dans lequel ladite étape de génération de signaux d'instruction comprend :

- la représentation des signaux électro-physiologiques acquis sur ladite au moins une fenêtre de temps d'observation sous la forme d'au moins un tenseur de données à N voies, N étant supérieur ou égal à un ; et
- la génération d'un signal de sortie correspondant à ladite au moins une fenêtre de temps d'observation en effectuant une régression sur ledit au moins un tenseur de données ;

**caractérisé en ce que** ledit procédé comprend une étape d'étalonnage selon l'une quelconque des revendications 1 à 7.

**9.** Procédé selon la revendication 8, dans lequel la génération dudit au moins un signal d'instruction a son propre rythme.

**10.** Procédé selon l'une quelconque des revendications 8 ou 9, comprenant la réalisation d'une régression partielle pénalisée des moindres carrés ou une régression partielle à multiples voies des moindres carrés, sur ledit tenseur de données à N voies.

**11.** Procédé selon l'une quelconque des revendications précédentes, dans lequel lesdits signaux électro-physiologiques sont des signaux électrocorticographiques (ECoG).

**Fig. 1**

1 point of observation - 3-way array
(3d order tensor)

**Fig.2**

**Fig. 3**

14

**Fig. 4**

**Fig. 5**

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEIGH R. HOCHBERG et al.** Neuronal ensemble control of prosthetic devices by a human with tetraplegia. *Nature,* 13 July 2006, vol. 442, 164-171
- **K. NAZARPOUR et al.** Parallel Space-Time-Frequency Decomposition of EEG Signals of Brain Computer Interfacing. *Proceedings of the 14th European Signal Processing Conference (EUSIPCO 2006),* 04 September 2006
- Multi-way Analysis in the Food Industry - Models, Algorithms, and Applications. **R. BRO.** PhD Thesis. University of Amsterdam, 1998
- **R. TIBSHIRANI.** Regression shrinkage and variable selection via lasso. *J. Roy. Statistic. Soc. Ser. B,* 1996, vol. 58, 267-288
- **R. TIBSHIRANI et al.** Sparsity and smoothness via the fused lasso. *R. Statist. Soc. B,* 2005, vol. 67, 91-108
- **H. ZOU ; T. HASTIE.** Regularization and variable selection via the elastic net. *J. Roy. Statistic. Soc. Ser. B,* vol. 67, 301-320

- **M. SCHMIDT.** Least Squares Optimization with L1-Norm Regularization. *Cs542B Project Report,* December 2005, http://www.di.ens.fr/~mschmidt/Software/lasso.pdf.
- **R. KOHAVI.** A study of cross-validation and bootstrap for accuracy estimation and model selection. *Proceedings of the Fourteenth International Joint Conference on Artificial Intelligence,* 1995, vol. 2 (12), 1137-1143
- **G. GOLUB et al.** Generalized cross-validation as a method for choosing a good ridge parameter. *Technometrics,* 1979, vol. 21, 215-223
- **H. AKAIKE.** A new look at the statistical model identification. *IEEE Trans. Automat. Control,* 1974, vol. 19, 716-723
- **G. SCHWARTZ.** Estimating the dimension of a model. *Ann. Statist.,* 1978, vol. 6, 461-464
- **R.D. COOK ; S. WEISBERG.** Residuals and Influence in Regression. Chapman and Hall, 1982